# EUROPEAN PATENT APPLICATION

(11) **EP 0 851 350 A2**
(43) Date of publication of application: **01.07.1998**
(21) Application number: 97113312.9
(22) Date of filing: 01.08.1997
(51) Int. Cl.: G06F 11/00

(54) **Programmable independent watchdog circuitry**

(30) Priority: 12.11.1996 US 747412
(71) Applicant: WaferScale Integration Inc., Fremont, CA 94528 (US)
(72) Inventor: The designation of the inventor has not yet been filed
(74) Representative: Grünecker, Kinkeldey, Stockmair & Schwanhäusser Anwaltssozietät

(57) **Abstract**

An independent watchdog circuit operating with a microcontroller includes a non-volatile memory element and a counter. The non-volatile memory element stores a counting value therein. The counter is connected to the non-volatile memory element and times an operation of the microcontroller, the length of the timing being based on the counting value stored in the non-volatile memory element. The circuit can include a pulse width circuit which defines the width of the reset pulse provided by the watchdog circuit. Also disclosed is a watchdog circuit operating with a programmable logic device.

## Description

### FIELD OF THE INVENTION

The present invention relates to watchdog circuitry in general.

### BACKGROUND OF THE INVENTION

Microcontrollers and microprocessors typically include watchdog circuitry which detect errors in the software running thereon by timing certain repetitive software operations. Through this timing, the watchdog circuitry helps the microcontroller or microprocessor controlling it to recover from disturbances that cause system malfunctions. Therefore, watchdog circuitry should be an extremely reliable unit performing its task at all times, even in hostile environments. Watchdog circuitry can be implemented internal to the microcontroller circuitry or as discrete, stand-alone devices.

The M68HC11 microcontroller of Motorola Inc. of the USA is an example of a microcontroller which operates with a watchdog circuit. The watchdog circuit is described in sections 5.2.2 and 10.2.3 of the HC11 reference manual.

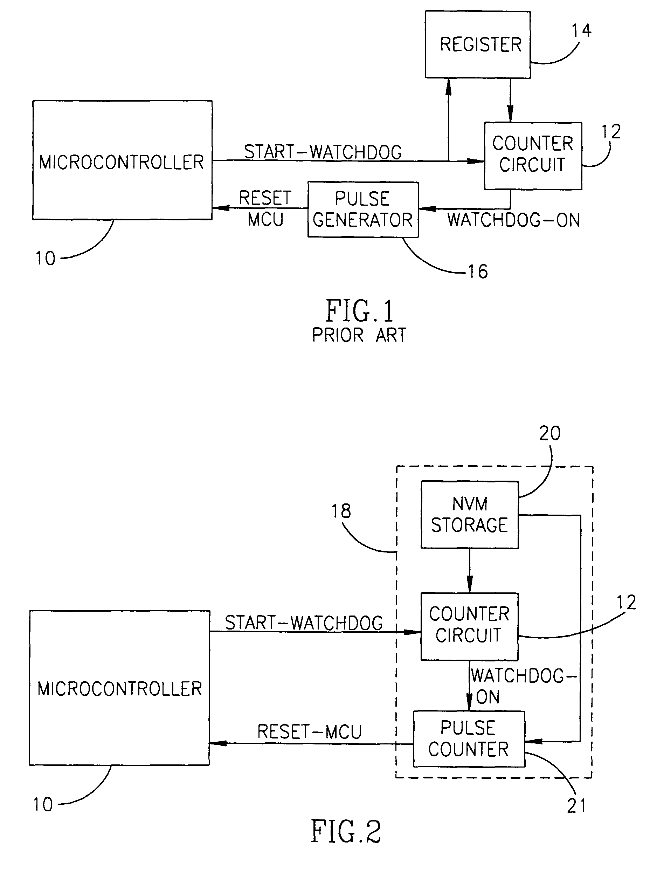

Prior art Fig. 1, to which reference is now made, shows a microcontroller 10 and a discrete watchdog circuit comprised of a counter circuit 12, a register 14 and a pulse generator 16. The microcontroller 10 stores a count value in register 14 and the counter circuit 12 measures time by counting down from or up to the value stored in register 14. At the same time, the repetitive software operation runs on the microcontroller 10. When the operation successfully ends, or arrives at an appropriate location in the software, the software indicates to the microcontroller 10 to provide a start-watchdog signal to the counter circuit 12, enabling the watchdog circuit to start the timing process again.

If, on the other hand, there is a malfunction in the software and the counter circuit 12 finishes counting before the microcontroller issues the start-watchdog signal, the counter circuit 12 sends a watchdog-ON signal to the pulse generator 16 which, in turn, generates a reset-microcontroller pulse to the microcontroller 10, thereby resetting the microcontroller 10. After reset, the microcontroller 10 reinitializes its system resources which, hopefully, causes the system to recover from the malfunction. The reset signal can be of any length and the pulse generator 16 can include a counter circuit to count the length of the pulse.

The length of the time to be measured is defined by the value of the variable stored in the register 14 and by the clock speed of the counter circuit 12. Typically, the microcontroller 10 stores the variable in the register 14 at some time shortly after power up. This works well as long as the power-up occurs smoothly. However, if there is a glitch at power-up, the variable may not be written correctly, it may not be written at all or it may be written too late. At this point, the counter circuit 12 will not work properly vis-a-vis regulating the timing of the software operation.

Furthermore, the counter circuit 12 accesses register 14 every time the start watchdog signal is issued. Therefore, it is necessary for the contents of register 14 not to change at any time. However, the content of register 14 is susceptible to any type of disturbances which might occur in the system, be it a power glitch, radio frequency (RF) interference, noise-induced crosstalk or microcontroller software bugs. Once the original content of register 14 changes, the watchdog circuit will not operate properly, making it impossible for the system to recover from those events which disturb the scheduled operation of the microcontroller.

In addition, counter circuit 12 can operate with a plurality of clocks, each having a different clock speed, where the selection of the clock speed is software dependent. Thus, the length of time to be measured may change even though the value to be counted does not change. Other software selections can include clock divisor, output waveform, etc. These selections typically involve storing the selected value in a register. The content of the registers is as susceptible to change as register 14, as described hereinabove.

### SUMMARY OF THE PRESENT INVENTION

Applicants have realized that the prior art implementations of watchdog circuits are susceptible to disturbances and, therefore, cannot reliably function to correct random errors to the system. If the watchdog circuit could operate independently, then, at power-up, the watchdog circuit could begin operating regardless of the state of the microcontroller and could continue to operate reliably during and after potentially harmful system events.

In the present invention, Applicants create the independent watchdog circuit by utilizing non-volatile memory elements which are programmed before the part is first placed in its target system and which retain their content and functionality thereafter.

There is therefore provided, in accordance with a preferred embodiment of the present invention, an independent watchdog circuit which includes a non-volatile memory element and a counter. The non-volatile memory element stores a counting value therein. The counter is connected to the non-volatile memory element and times an operation of the microcontroller, the length of the timing being based on the counting value stored in the non-volatile memory element.

Additionally, in accordance with a preferred embodiment of the present invention, the non-volatile memory element can be an ultraviolet (UV) erasable programmable read only memory (EPROM) or an electrically erasable programmable read only memory (EEPROM).

Moreover, in accordance with a preferred embodiment of the present invention, the circuit can include a pulse width value stored in the non-volatile memory element and a pulse counter which receives a start signal from the watchdog counter and times the length of a reset signal to the microcontroller.

Further, in accordance with a preferred embodiment of the present invention, the circuit can include a programmable logic device (PLD) which receives a plurality of input signals from the microcontroller and provides a start-watchdog signal to the watchdog counter as a function of at least some of the input signals. The PLD also receives a watchdog-on signal at least from the watchdog counter and provides a reset signal to the microcontroller as a function of at least the watchdog-on signal. The PLD functionality is also determined by non-volatile memory elements.

### BRIEF DESCRIPTION OF THE DRAWINGS

The present invention will be understood and appreciated more fully from the following detailed description taken in conjunction with the drawings in which:
Fig. 1 is a schematic circuit diagram illustration of a prior art watchdog circuit;
Fig. 2 is a schematic circuit diagram illustration of an independent watchdog circuit, constructed and operative in accordance with a preferred embodiment of the present invention; and
Fig. 3 is a circuit diagram illustration of an alternative independent watchdog circuit, constructed and operative in accordance with a second preferred embodiment of the present invention.

### DETAILED DESCRIPTION OF PREFERRED EMBODIMENTS

Reference is now made to Fig. 2 which illustrates an independent watchdog circuit, constructed and operative in accordance with a preferred embodiment of the present invention. Reference numerals in Fig. 2 which are similar to those of Fig. 1 refer to similar elements.

The system of Fig. 2 comprises microcontroller 10 and an independent watchdog circuit 18 comprising counter circuit 12, a pulse counter 21 and, in accordance with a preferred embodiment of the present invention, a non-volatile memory (NVM) element 20, such as an ultraviolet (UV) erasable programmable read only memory (EPROM) or an electrically erasable programmable read only memory (EEPROM), for storing the counting value of the counter circuit 12 and any other configuration data, such as clock speed, etc.

All of the configuration data, and especially, the counting value, are stored in the NVM element 20 at the time of programming. Because NVM elements are non-volatile, they generally will not change in the presence of electrical or other disturbances. Therefore, the data is generally always available. Thus, at power up of the microcontroller 10, there is no need to enter the counting value into the element 20 since the value is already present therein. Accordingly, the counter circuit 12 can begin counting and the microcontroller 10 can begin performing the operation to be timed as soon as full power is present. Furthermore, the NVM element 20 ensures that all of the counter circuit parameters, such as timing length, clock source, clock divisor or output waveform format, are consistent throughout the operation of the watchdog circuit. The NVM element 20 enables the watchdog circuit 18 to be independent of the microcontroller 10 or of any source of disturbance.

The system of the present invention operates as follows:

The counter circuit 12 starts counting down (or up) after the count value is initially loaded from the NVM element 20. Under normal software operating conditions, the microcontroller 10 periodically arrives at an appropriate code location where the software indicates to microcontroller 10 to issue a start-watchdog signal to counter circuit 12. This, in turn, causes counter circuit 12 to reload its count value from NVM element 20 and to resume counting from (or to) the initial value.

As long as counter circuit 12 is restarted before it completes counting down to zero or up to the count value, its output signal is not active. However, if there is an error in the software execution, counter circuit 12 finishes counting and sets a watchdog-on signal to its active state.

The active duration of the watchdog-on pulse is determined by a pulse generator as in the prior art. However, in accordance with a preferred embodiment of the present invention, the pulse generator of the present invention comprises pulse counter 21 which operates with data stored in NVM element 20. The length of the pulse is determined by NVM data. The output of pulse counter 21 is the reset signal to microcontroller 10, enabling it to reinitialize the system and setting it back to the correct operating mode.

It will be appreciated that, since the count values for counters 12 and 21 and any other configuration data are stored in NVM element 20, their values are not likely to change as a result of a disturbance. Therefore, the watchdog circuit of the present invention is generally returned to its original state and thus, can generally consistently reset the microcontroller 10.

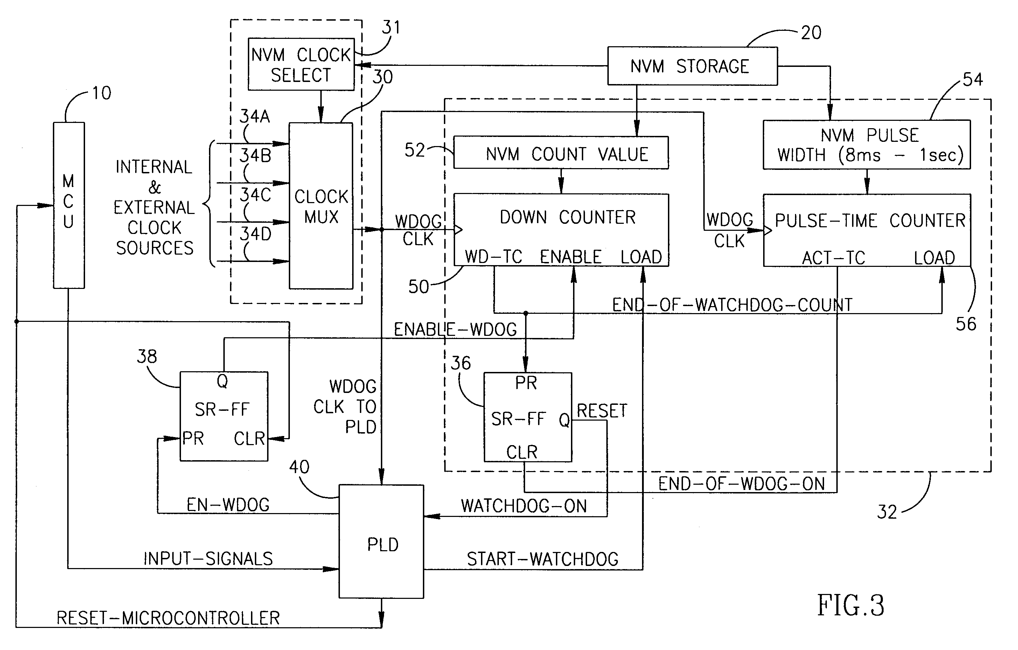

Reference is now made to Fig. 3 which illustrates the present invention in more detail and provides a further embodiment of the present invention which operates also in conjunction with a programmable logic device (PLD) 40. As is known in the art, PLDs implement logic functions on the signals provided thereto. In the present embodiment, at least one of the logic functions of interest is the response to a watchdog-on signal. Since the logic functions of the PLD are "burned in" and are not easily changeable, PLDs are also non-volatile elements and thus, the response to the software error in the present embodiment will generally always be the same.

The circuit of Fig. 3 comprises the microcontroller 10, a clock multiplexer 30, a watchdog circuit 32, NVM storage element 20 and PLD 40.

Watchdog circuit 32 is an independent watchdog circuit and comprises a down counter 50 operating with an NVM count value 52, a pulse counter 56 operating with an NVM pulse width value 54 and two set-reset flip-flops 36 and 38. NVM values 52 and 54 are stored in NVM element 20. Both the down counter 50 and the pulse counter 56 count in accordance with the watchdog-clock signal. The down counter 50 has enable and load inputs and a watchdog terminal count (WDTC) output and the pulse counter 56 has a load input and an active terminal count output.

The clock multiplexer 30 is connected to a plurality of clock lines 32, for example 32A, 32B, 32C and 32D, each having a different clock signal thereon. The clock signals can be from external or internal clock sources. Systems without dependable external clock sources can utilize free-running internal oscillators, if such are present. Since the latter are internal, they will generally operate in harsh conditions, thus ensuring that the watchdog circuit also operates during such conditions.

The clock multiplexer 30 selects the clock rate for the watchdog circuit 32 according to a clock select value 31 stored in the NVM element 20. Clock multiplexer 30 provides the watchdog-clock signal to the watchdog circuit 32 and to the PLD 40.

As in prior art PLDs, PLD 40 receives input signals from the microcontroller 10 and comprises a plurality of user-defined logic functions which implement user-defined commands on the input signals and produce product term signals as a result. The commands can be of any type and can also define a command sequence. In accordance with a preferred embodiment of the present invention, PLD 40 has three product term signals which affect the watchdog circuit, the start-watchdog signal, the en-wdog signal and the reset-microcontroller signal. Thus, in the present invention, PLD 40 implements a command or series of commands by which it determines how to control or respond to watchdog circuit 32.

PLD 40 will activate the start-watchdog signal, provided to the load input of down counter 50, only if the relevant command sequence has executed before down counter 50 finishes counting. The command sequence in the PLD 40 activates the en-wdog signal, provided to the flip-flop 38, after power up and disables the en-wdog signal whenever the PLD 40 issues the reset-microcontroller signal. PLD 40 reactivates the en-wdog signal after the microcontroller 10 has been reset and the relevant command sequence has been executed. The PLD 40 activates the reset-microcontroller signal after receiving a wdog-on signal from flip-flop 36 (described in detail hereinbelow) and after performing the relevant command sequence, as programmed therein by the user.

PLD 40 provides the en-wdog signal to the preset (PR) input of flip-flop 38 which, in response to a logic one signal thereon, provides an enable-watchdog signal to the enable input of down counter 50, thereby enabling the activity of down counter 50. PLD 40 provides the reset-microcontroller to the clear (CLR) input of flip-flop 38 and thus, when the reset-microcontroller signal is activated, flip-flop 38 disables the enable-watchdog signal.

Watchdog circuit 32 operates similar to that described for the first embodiment, loading NVM count value 52 upon receipt of the start-watchdog signal from PLD 40 and providing an end-of-watchdog-count (also known as the watchdog terminal count (WDTC)) signal when it has finished counting down from NVM count value 52.

The end-of-watchdog-count signal is provided to flip-flop 36 which, in response, changes to the active "1" state and provides an active watchdog-on signal, via its output Q, to the PLD 40. Thus, when the flip-flop 36 is activated, it provides the watchdog-on signal to the PLD 40. has enable and load inputs and a watchdog terminal count (WDTC) output and the pulse counter 56 has a load input and an active terminal count output.

The clock multiplexer 30 is connected to a plurality of clock lines 32, for example 32A, 32B, 32C and 32D, each having a different clock signal thereon. The clock signals can be from external or internal clock sources. Systems without dependable external clock sources can utilize free-running internal oscillators, if such are present. Since the latter are internal, they will generally operate in harsh conditions, thus ensuring that the watchdog circuit also operates during such conditions.

The clock multiplexer 30 selects the clock rate for the watchdog circuit 32 according to a clock select value 31 stored in the NVM element 20. Clock multiplexer 30 provides the watchdog-clock signal to the watchdog circuit 32 and to the PLD 40.

As in prior art PLDs, PLD 40 receives input signals from the microcontroller 10 and comprises a plurality of user-defined logic functions which implement user-defined commands on the input signals and produce product term signals as a result. The commands can be of any type and can also define a command sequence. In accordance with a preferred embodiment of the present invention, PLD 40 has three product term signals which affect the watchdog circuit, the start-watchdog signal, the en-wdog signal and the reset-microcontroller signal. Thus, in the present invention, PLD 40 implements a command or series of commands by which it determines how to control or respond to watchdog circuit 32.

PLD 40 will activate the start-watchdog signal, provided to the load input of down counter 50, only if the relevant command sequence has executed before down counter 50 finishes counting. The command sequence in the PLD 40 activates the en-wdog signal, provided to the flip-flop 38, after power up and disables the en-wdog signal whenever the PLD 40 issues the reset-microcontroller signal. PLD 40 reactivates the en-wdog signal after the microcontroller 10 has been reset and the relevant command sequence has been executed. The PLD 40 activates the reset-microcontroller signal after receiving a wdog-on signal from flip-flop 36 (described in detail hereinbelow) and after performing the relevant command sequence, as programmed therein by the user.

PLD 40 provides the en-wdog signal to the preset (PR) input of flip-flop 38 which, in response to a logic one signal thereon, provides an enable-watchdog signal to the enable input of down counter 50, thereby enabling the activity of down counter 50. PLD 40 provides the reset-microcontroller to the clear (CLR) input of flip-flop 38 and thus, when the reset-microcontroller signal is activated, flip-flop 38 disables the enable-watchdog signal.

Watchdog circuit 32 operates similar to that described for the first embodiment, loading NVM count value 52 upon receipt of the start-watchdog signal from PLD 40 and providing an end-of-watchdog-count (also known as the watchdog terminal count (WDTC)) signal when it has finished counting down from NVM count value 52.

The end-of-watchdog-count signal is provided to flip-flop 36 which, in response, changes to the active "1" state and provides an active watchdog-on signal, via its output Q, to the PLD 40. Thus, when the flip-flop 36 is activated, it provides the watchdog-on signal to the PLD 40.

As mentioned hereinabove and in accordance with a preferred embodiment of the present invention, the watchdog-on signal to the PLD 40 can also be utilized as an input to the PLD 40, allowing the circuit designer to program a suitable response to the situation where the watchdog circuit 32 has fully counted down. One suitable response may be to close down other active circuits in anticipation of the resetting of the microcontroller 10.

The end-of-watchdog-count signal is also provided to the load input of pulse counter 56 which, in response, loads the NVM pulse width value 54. With value 54, pulse counter 56 determines the length of the watchdog-on pulse, activating an end-of-watchdog-on signal when the count has finished. Since the end-of-watchdog-on line is connected to the clear CLR input of flip-flop 36, activation of the end-of-watchdog-on signal deactivates the watchdog-on signal. Thus, once the pulse counter 56 has finished counting, the flip-flop 36 is cleared and the watchdog-on signal which the flip-flop 36 produces is returned to the inactive "0" state.

It will be appreciated that the PLD-based circuit of Fig. 3 can also be implemented with prior art watchdog circuits wherein the storage elements are not non-volatile elements but rather, writable storage elements.

It will be appreciated by persons skilled in the art that the present invention is not limited to what has been particularly shown and described hereinabove. Rather the scope of the present invention is defined only by the claims which follow:

## Claims

1. An independent watchdog circuit operating with a microcontroller, the watchdog circuit comprising:
a. a non-volatile memory element storing at least a counting value therein; and
b. a watchdog counter connected to said non-volatile memory element for timing an operation of said microcontroller, the length of the timing being based on said counting value stored in said non-volatile memory element.

2. A circuit according to claim 1 and wherein said non-volatile memory element is one of the following group: an ultraviolet (UV) erasable programmable read only memory (EPROM) and an electrically erasable programmable read only memory (EEPROM).

3. A circuit according to claim 1 and also comprising a pulse width value stored in said non-volatile memory element and a pulse counter for receiving a start signal from said watchdog counter and for timing the length of a reset signal to said microcontroller.

4. A circuit according to claim 1 and also comprising a clock multiplexer for selecting among a plurality of clock signals and wherein said non-volatile memory element additionally stores configuration data for said clock multiplexer.

5. A circuit according to claim 1 and also comprising a programmable logic device for receiving a plurality of input signals from said microcontroller, for providing at least a start-watchdog signal to said watchdog counter as a function of at least some of said input signals, for receiving a watchdog-on signal at least from said watchdog counter and for providing a reset signal to said microcontroller as a function of at least said watchdog-on signal.

6. A circuit according to claim 5 and also comprising an enable flip-flop for producing a watchdog counter enable/disable signal from said reset signal and an enabling signal produced by said programmable logic device.

7. A pulse width circuit operative with a watchdog circuit for defining the pulse width of a terminal count output from the watchdog circuit, the watchdog circuit having at least a terminal count output and a reset input, the pulse width circuit comprising:
a. a flip-flop having preset and clear input ports and an output port, wherein said preset input port is connected to the terminal count output port of said watchdog circuit for changing to the active state when said terminal count output is active;
b. a non-volatile memory element storing a counting value therein; and
c. a counter connected to said non-volatile memory element, to said terminal count output port of said watchdog circuit and to said clear input port of said flip-flop for defining the time interval during which said flip-flop is in the active state by counting up to or down from said counting value and by clearing the flip-flop to the inactive state when said counting is finished.

8. A circuit comprising:
a. a microcontroller;
b. a programmable logic device connected on input to a set of input signals from said microcontroller and connected on output to at least a start-watchdog line and a reset microcontroller line, said reset microcontroller line being connected to said microcontroller; and
c. a watchdog circuit connected on input to at least said start-watchdog line and on output to a terminal count line connected to said microcontroller and said programmable logic device, for timing an operation of said microcontroller and for providing a terminal count signal on said terminal count line when said operation is not finished within a predetermined length of time.

9. A circuit according to claim 8 and also comprising a pulse width circuit operative with a watchdog circuit for defining the pulse width of said terminal count output signal from said watchdog circuit.

10. A circuit according to claim 9 and wherein said pulse width circuit comprises:
a. a flip-flop having preset and clear input ports and an output port, wherein said preset input port is connected to the terminal count output port of said watchdog circuit for changing to the active state when said terminal count output is active;
b. a non-volatile memory element storing a counting value therein; and
c. a counter connected to said non-volatile memory element, to said terminal count output port of said watchdog circuit and to said clear input port of said flip-flop for defining the time interval during which said flip-flop is in the active state by counting up to or down from said counting value and by clearing the flip-flop to the inactive state when said counting is finished.

11. A circuit according to claim 8 and also comprising an enable flip-flop having preset and clear input ports and an output port, wherein said preset input port is connected to an enabling signal produced by said programmable logic device, said clear input port is connected to said reset microcontroller line and said output port is connected to a watchdog counter enable/disable line which is connected to an enable input of said watchdog counter.
